# EUROPÄISCHE PATENTSCHRIFT

(11) **EP 0 480 472 B1**
(45) Veröffentlichungstag und Bekanntmachung des Hinweises auf die Patenterteilung: **28.02.1996**
(21) Anmeldenummer: 91117449.8
(22) Anmeldetag: 12.10.1991
(51) Int. Cl.: A61K 6/083, C08F 2/46

(54) **Durch Einwirkung von Schwingungen herstellbare und verarbeitbare Dentalmassen und Verfahren zu ihrer Herstellung**
Dental composition which can be prepared and processed under vibration, and method for its preparation
Composition dentaire pouvant être préparée et façonnée sous vibrations et son procédé de préparation

(30) Priorität: 12.10.1990 DE 4032505; 18.02.1991 DE 4104934
(43) Veröffentlichungstag der Anmeldung: 15.04.1992
(73) Patentinhaber: THERA Patent GmbH & Co. KG Gesellschaft für industrielle Schutzrechte, D-82229 Seefeld (DE)
(72) Erfinder: Eidenbenz, Stefan, CH-8001 Zurich (CH); Ellrich, Klaus, Dr., W-8031 Wörthsee-Steinebach (DE); Gasser, Oswald, Dr., W-8031 Seefeld (DE); Guggenberger, Rainer, Dr., W-8036 Herrsching (DE); Iburg, Andreas, W-8031 Wörthsee (DE); Koran, Peter, Dr., W-8120 Weilheim (DE); Noack, Michael J., Dr., W-1000 Berlin 28 (DE); Nowak, Reinhold, Dr., W-8081 Adelshofen (DE); Roulet, François, Prof., W-1000 Berlin 19 (DE); Stefan, Klaus-Peter, Dr., W-8031 Seefeld (DE); Zöllner, Werner, Dr., W-8031 Wörthsee-Steinebach (DE)
(74) Vertreter: Müller, Bernhard, Dr. H. Schmidt & B. Müller Patentanwälte

(56) Entgegenhaltungen:
- EP-A- 0 091 990
- EP-A- 0 100 474
- EP-A- 0 113 926
- EP-A- 0 325 266
- GB-A- 2 201 093
- US-A- 4 219 619
- DEUTSCHE ZAHNÄRTZLICHE ZEITUNG Bd. 33, Nr. 3, März 1978, Seiten 199 - 203; H.J. SCHRÖDER E.A.: 'VERÄNDERUNGEN DES FÜLLUNGSGRANDES DURCH ULTRASCHALL'

## Beschreibung

Die Erfindung betrifft Verfahren zur Herstellung von Dentalmassen und und insbesondere Verfahren zur Herstellung und zur Verwendung von hochgefüllten dentalen Füllmaterialien (Füllungscompositen) zur Befestigung von Inlays, Onlays, Verblendschalen und dergl. mittels hochfrequenter Vibrationen. Als derartige Massen kommen insbesondere dentale Präparate, z.B. Füllmassen, aber auch andere Massen, wie Klebstoffe und Spachtelmassen, in Frage.

Auf vielen technischen Gebieten, z.B. auf dem Gebiet der Dentalmassen sowie der Kleb- und Spachtelmassen, werden hochgefüllte viskose erhärtbare Massen eingesetzt, um Defekte auszufüllen oder defekte Teile miteinander zu verbinden. Die Haltbarkeit dieser Restaurationen wird im wesentlichen von den Eigenschaften der Binder und Füllstoffe bestimmt, wobei im allgemeinen der Binder den schwächeren Teil darstellt. Es hat sich deswegen gezeigt, daß die Massen besonders dauerhafte und hochwertige Restaurationen immer dann erlauben, wenn der Füllstoffgehalt sehr hoch ist. So werden auf dem Dentalgebiet Zahnfüllmassen mit bis zu 85 Gew.-% Füllstoffen angeboten, Zahnzemente weisen üblicherweise 50 bis 60 Gew.-% Füllstoffe auf.

Diese Aufnahme an Füllstoffen ist vor allem durch die für den Anwender notwendige Viskositätseinstellung begrenzt. So müssen z.B. Zahnfüllmassen noch so plastisch verformbar sein, daß der Zahnarzt sie mit geeigneten Instrumenten, wie z.B. Spateln und Kugelstopfern, in die Kavität einfüllen kann, so daß diese unter Druck so weit verflüssigbar sind, daß sie die Kavität vollständig ausfließen und mit den Instrumenten auch noch so bearbeitbar sind, daß die Oberfläche der natürlichen Gegebenheit angepaßt werden kann. Zur Befestigung von Inlays, Onlays, Verblendschalen und dergl. werden üblicherweise leichtfließende Zementmassen verwendet, welche im allgemeinen einen niedrigen Gehalt an anorganischen Füllstoffen besitzen. Solche Zementmassen erhärten entweder durch Ionenreaktionen, z. B. sogenannte Zinkphosphat- oder Glasionomerzemente, oder aber durch Polymerisationsreaktionen, wie z. B. durch radikalische Polymerisation von Methacrylsäureestern.

Diese letztgenannten Materialien werden vor allem eingesetzt, wenn es sich um hinreichend transparente Inlays, Onlays sowie Verblendschalen, z. B. aus Porzellan oder Kunststoff, handelt. Standardmäßig werden hierzu vorzugsweise lichthärtende Materialien eingesetzt, wobei zum Teil über einen nachgeschalteten Redox-Prozeß noch eine Nachhärtung erfolgt.

Die europäische Patentanmeldung 0 325 266 beschreibt beispielsweise Dentalmaterialien, die auch zum Zementieren von Inlays, Onlays sowie Verblendschalen verwendet werden können, die in zwei Schritten gehärtet werden. Hierzu enthalten die Massen zwei unterschiedliche Photoinitiatoren mit unterschiedlichen Lichtabsorptionen. In dieser Patentschrift sind zwar hochgefüllte Zahnfüllmassen beschrieben, aber Zementierungsmassen, die nach diesem System hergestellt werden, haben üblicherweise weniger als 50 Gew.-% Füllstoffe.

Noch anspruchsvoller sind die Anforderungen auf dem Gebiet der Zementierungsmaterialien und Klebstoffe. Nachdem hier oft geringste Spalten mit den Klebstoffen ausgefüllt werden sollen, ist eine erhöhte Fließfähigkeit unter Druck notwendig. Üblicherweise sollten hier Filmstärken <25 µm unter geringem Druck erreicht werden. Es war deswegen bisher nicht möglich, Massen herzustellen und zu verwenden, die so hoch viskos sind und dabei so hoch mit Füllkörpern angefüllt sind, daß sie für den Anwender noch vernünftig verwendbar sind.

Herkömmlicherweise werden die meisten mehrkomponentigen Präparate mit einem Spatel auf einer Unterlage, z.B. einem Block vermischt. Hierbei werden also geeignete Volumina oder im Mischungsverhältnis vordosierte Volumina der einzelnen Komponenten auf einen Block gegeben und anschließend mit einem Spatel vermischt. Diese Vorgehensweise funktioniert bisher allerdings nur mit relativ niedrigviskosen oder hochthixotropen Materialien, die sich durch die Mischbewegung so stark verflüssigen, daß eine vollständige Vermischung hiermit gewährleistet ist. Bei weniger thixotropen und sehr hoch gefüllten Materialien ist es unmöglich, diese Scherkräfte überhaupt aufzubringen.

Weiterhin ist es bekannt, vordosierte, mehrkomponentige Materialien in Kapseln zu mischen (z.B. EP 0 157 121). Hierbei handelt es sich in aller Regel um ein Pulver/Flüssigkeitssystem, wobei im Kapselinnenkörper das Pulver, z.B. ein Glasionomerzementpulver gelagert ist und in einem außen an diesem Behälter angebrachten Kissen eine flüssige Komponente, die vor der Mischung durch ein spezielles Aktivatorsystem eingespritzt wird. Diese Kapseln werden anschließend bei hochfrequenten Schwingungen mit Amplituden >5 mm in der Kapsel gemischt. Der Nachteil bei diesem System ist allerdings, daß der Energieübertrag doch relativ schlecht ist, und daß bei den Mischbewegungen in der Kammer häufig etwas Luft mit eingebracht wird.

Aus der EP 232 733 ist es bekannt, niedrig- bis mittelviskose Massen (dentale Abformmassen oder Epoxidklebstoffe) mittels sogenannter statischer Mischelemente miteinander homogen zu vermischen. Hierbei werden die Materialien durch eine Kanüle, in die eine Mischwendel eingefügt ist, durchgepreßt.

US-PS 4 219 619 beschreibt ein vibrierendes Dentalinstrument zum Einsetzen von Kronen und Brücken. Hierbei wird durch relativ niederfrequente Vibration im Bereich 20 - 100 Hz ein üblicher, niederviskoser Zement beim Einsetzen der Krone mittels der Übertragungskette Vibrator-Bißregistrat-Krone verflüssigt. Eine Verflüssigung von hochviskosen Massen ist hierbei nicht beschrieben. Auch eine Verwendung von Füllungscompositen oder Compositezementen ist nicht beschrieben. Zudem erfordert das Verfahren ein spezielles Instrument, welches vom Zahnarzt extra angeschafft werden muß.

GB-A-2 201 093 beschreibt die Anwendung von Ultraschall auf dentale Füllmaterialien, die die Konsistenz eines üblichen lichthärtenden Comosites aufweisen. Die Verwendung von Materialien, die aufgrund ihrer Konsistenz nicht als Dentalmaterialien geeignet sind, wird in dieser Druckschrift nicht beschrieben.

Es stand also bisher kein Verfahren zur Verfügung, mit dem mehrkomponentige, hochgefüllte, hochviskose Massen beim Anwender (also per Hand oder in einer Mischkapsel) gleichmäßig vermischt werden können.

Aufgabe der Erfindung ist es, die vorgenannten Probleme dadurch zu lösen, daß dem Anwender extrem hochgefüllte und hochviskose Massen zur Verfügung gestellt werden, die mit neuartigen Herstellungsverfahren hergestellt werden können und mit neuartigen Verfahren zur Anwendung gebracht werden können.

Das Wesen der Erfindung ist darin zu sehen, daß hochgefüllte Massen, deren Viskosität so hoch ist, daß sie einer Bearbeitung mit den üblichen Methoden nicht zugänglich sind, durch eine Vibrationsbehandlung so verflüssigt werden, daß sie bestimmungsgemäß hergestellt, verarbeitet und verwendet werden können.

Gegenstand der Erfindung ist das in Anspruch 1 definierte Verfahren zur Herstellung von Dentalmassen.

Gegenstand der Erfindung ist das in Anspruch 2 definierte Verfahren zum Vermischen von mehrkomponentigen, hochgefüllten, hochviskosen Massen.

Gegenstand der Erfindung ist ferner die Verwendung von hochgefüllten dentalen Füllmaterialien zur Befestigung von Inlays, Onlays, Verblendschalen oder dergl. mittels hochfrequenter Vibrationen.

Vorzugsweise wird die Vibration mittels eines Ultraschallgerätes erzeugt.

Der erfindungsgemäße Frequenzbereich beträgt vorzugsweise 50 Hz bis 50 kHz und insbesondere 100 Hz - 30 kHz. Die bevorzugten Amplituden sind 20 µm bis 2 mm und insbesondere50 µm bis 1 mm.

Mit dem erfindungsgemäßen Verfahren lassen sich erstmals auch Massen mischen, die eine Viskosität aufweisen, die so hoch ist, daß die Massen mit bekannten Methoden beim Anwender (also per Hand oder in einer Mischkapsel) nicht mehr vermischt werden können. Solche Massen in mehrkomponentiger Form sind insbesondere immer dann vorteilhaft, wenn man mehrere verschiedene Aushärtungsmechanismen miteinander kombinieren möchte. Insbesondere vorteilhaft ist es auf dem Gebiet der Photohärtung in Schattenbereichen. Hierbei kann man noch einen zweiten Härtungsmechanismus, der unabhängig von der Beleuchtungsquelle ist, mit einbauen, wie z. B. ein Redox-Initiator-System für Methacrylate. Dieses besteht beispielsweise aus Peroxiden, wie Benzoylperoxid, und Aktivatoren, z. B. Amine, insbesondere aromatische Amine oder andere Reduktionsmittel wie Barbitursäuren, oder deren Derivate oder aber Malonylsulfonamide und deren Derivate.

Für die erfindungsgemäß anzumischenden Dentalmassen kommen ethylenisch ungesättigte Monomere bzw. Polymere in Frage, z.B. monomere und polymere Acrylate und Methacrylate. Hingewiesen sei diesbezüglich auf die in der DE-OS 36 09 038 beschriebenen Massen, wobei die dort beschriebenen röntgenopaken Füllstoffe auch entfallen können.

Als ethylenisch ungesättigte Monomere bzw. Polymere für Dentalmassen seien beispielsweise die monomeren und polymeren Acrylate und insbesondere Methacrylate hervorgehoben. Bei polymerisierbaren Dentalmassen verwendet man insbesondere oft die langkettigen Monomere der US-PS 3,066,112 auf der Basis von Bisphenol-A und Glycidylmethacrylat oder dessen durch Addition von Isocyanaten erhaltenen Derivate. Besonders geeignet sind auch die Acrylsäure- bzw. Methacrylsäureester ein- oder vorzugsweise mehrwertiger Alkohole, beispielsweise Triethylenglycoldimethacrylat u. ä. Besonders geeignet sind auch die in der DE-PS 28 16 823 genannten Diacryl- und Dimethacrylsäureester des Bishydroxymethyltricyclo-(5.2.1.0^{2,6})-decans. Verwendet werden können auch die Reaktionsprodukte aus Diisocyanaten und Hydroxyalkyl(meth)acrylaten, wie beispielsweise in der DE-OS 23 12 559 beschrieben. Selbstverständlich können auch Gemische aus geeigneten Monomeren bzw. hieraus hergestellte ungesättigte Polymere verwendet werden.

Als Photoinitiatoren können alle Substanzen eingesetzt werden, die nach Bestrahlen durch UV- oder sichtbares Licht die Polymerisation auslösen, beispielsweise Benzoinalkylether, Benzilketale, Acylphoshinoxide oder aliphatische und aromatische 1,2-Diketonverbindungen, z. B. Campherchinon, wobei die Lichtpolymerisation durch Zusatz von Aktivatoren, wie tertiären Aminen oder organische Phosphiten, in an sich bekannter Weise beschleunigt werden kann.

Geeignete Initiatorsysteme zur Auslösung der Polymerisation über einen Redox-Mechanismus sind beispielsweise die Systeme Peroxid/Amin oder Peroxid/Barbitursäurederivate u. dergl. Bei Verwendung solcher Initiatorsysteme ist es zweckmäßig, einen Initiator (z. B. Peroxid) und eine Katalysatorkomponente (z. B. Amin) getrennt bereitzuhalten. Die beiden Komponenten werden dann kurz vor der Anwendung miteinander homogen vermischt.

Das erfindungsgemäße Verfahren läßt sich aber auch vorteilhaft für andere mehrkomponentige und hochviskose Massen verwenden, wie zum Anmischen hochviskoser Abformmassen oder Zahnzemente, wie Glasionomerzemente oder Zink/Phosphatzemente.

Auch auf dem Gebiet der mehrkomponentigen Klebstoffe und Spachtelmassen eröffnet sich ein neues Anwendungsgebiet durch die Verwendung hochgefüllter, hochviskoser Massen, die sich bisher nicht vernünftig mischen ließen.

Bevorzugt sind allerdings Verfahren zum Vermischen von dentalen Präparaten.

Es hat sich überraschenderweise gezeigt, daß durch die Verwendung von hochfrequenten Vibrationen bei der Befestigung von Inlays, Onlays sowie Verblendschalen auch hochviskose, hochgefüllte Massen so leichtfließend werden, daß die Benetzung des Ersatzteils sowie der verbleibenden Zahnhartsubstanz optimal ist und minimale Filmstärken des Zementierungscomposites erzielt werden können.

Unter hochfrequenten Vibrationen werden hierbei solche mit mehr als 200 Hz verstanden. Frequenzen mit mehr als 1 MHz sind nicht mehr zur Zementierung geeignet.

Vorzugsweise werden hochfrequente Vibrationen mit mehr als 1000 Hz, bevorzugt mehr als 5000 Hz, besonders bevorzugt mehr als 10 000 Hz, verwendet.

Geeigneterweise werden die hochfrequenten Vibrationen mit sogenannten "Sonic Scalern" und/oder "Ultrasonic Scalern" zur Befestigung angewandt. Solche Geräte werden in der Zahnarztpraxis zum Entfernen von Zahnstein sowie Füllungsüberschüssen bereits lange verwendet. Beim Aufbringen des Ultraschalls wird hierzu gleichzeitig über eine Wasserzufuhr eine Kühlung erreicht. Bei der erfindungsgemäßen Verwendung ist es vorteilhaft, diese Wasserkühlung abzustellen, mit dem gerundeten Scaler-Mittelteil auf die Oberfläche des Inlays, Onlays oder der Verblendschale mit geringem Druck einzuwirken, so daß dieses in die mit Composite aufgefüllte Kavität einsinken kann.

Hierbei ist es vorteilhaft, zwischen Ultraschallgerät und z. B. Inlayoberfläche eine Zwischenschicht, z. B. Papier, Wachspapier o. ä., anzubringen, um eine Verletzung der Inlayoberfläche über den Energieeintrag des Ultraschallgerätes zu vermeiden. Dieses kann auch in einer günstigen Ausführungsform dadurch geschehen, daß über den Scaler-Mittelteil eine Kunststoffhülse geführt wird. Die Verwendung von hochelastischen, gummiartigen Materialien, wie z. B. Bißregistrierungsmaterialien, ist hierzu nicht geeignet, da diese die hochfrequenten Vibrationen nicht mehr übertragen sondern absorbieren.

Die Composite-Füllmaterialien enthalten vorzugsweise folgende Komponenten:
a) 60 - 95, vorzugsweise 70 - 90, Gew.-% anorganische Füllstoffe;
b) 4 - 39,99, vorzugsweise 9 - 29,9, Gew.-% ethylenisch ungesättigte polymerisierbare Monomere und/oder Polymere;
c) 0,01 - 3, vorzugsweise 0,1 - 2, Gew.-% Photoinitiatoren;
d) sowie ggf. Aktivatoren, Initiatoren für die Initiierung einer Redox-Polymerisation sowie Pigmente, röntgenopake Zusatzstoffe und/oder Thixothopiehilfsmittel.

Die Füllkörper haben vorzugsweise eine mittlere Kornverteilung <20 µm und insbesondere <5 µm sowie eine obere Korngrenze von 150, vorzugsweise 70 µm und insbesondere <25 µm. Besonders vorteilhaft werden sogenannte Hybridcomposite verwendet, welche 5 - 25 Gew.-% Füllstoffe mit einer mittleren Korngröße von 0,02 - 0,06 µm und 65 - 85 Gew.-% Füllkörper mit einer mittleren Korngröße von 1 - 5 µm enthalten. Anorganische Füllstoffe können beispielsweise Quarz, gemahlene Gläser, Kieselgele sowie pyrogene Kieselsäuren oder deren Granulate sein. Besonders bevorzugt werden röntgenopake Füllstoffe, zumindest teilweise, mit eingesetzt. Diese können zum einen röntgenopake Gläser sein, also Gläser, welche z. B. Strontium, Barium oder Lanthan enthalten, oder ein Teil der Füllkörper besteht aus einem röntgenopaken Zusatz, wie beispielsweise Yttriumfluorid, Strontiumhexafluorozirkonat oder Fluoriden der Selten-Erdmetalle.

Zum besseren Einbau in die Polymermatrix ist es von Vorteil, die anorganischen Füllstoffe zu hydrophobieren. Übliche Hydrophobierungsmittel sind Silane, beispielsweise Trimethoxymethacryloyloxypropylsilan.

Der Füllstoffanteil in den anzumischenden Massen kann beispielsweise 60 bis 95 Gew.-% betragen, wobei für dentale Präparate Füllstoffanteile von 80 bis 95 Gew.-% und für Klebstoffe und Spachtelmassen von 60 bis 80 Gew.-% von besonderem Interesse sind.

Zur Herstellung der Massen mit hohem Füllstoffanteil bedient man sich beispielsweise herkömmlicher Knettaggregate, an die eine Schwingung im Frequenzbereich von 20 Hz bis 20 MHz mit einer Amplitude von 1 µm bis 5 mm angelegt wird. Durch die angelegte Schwingung werden die Komponenten während der Herstellungsprozedur hierbei so weit flüssig gehalten, daß ein deutlich erhöhter Füllstoffanteil resultiert. Nach Abstellen der Schwingung ist das Material dann so hochviskos, daß es mit den üblichen Anwendungen nicht mehr verarbeitbar ist. Es kann dann nur durch erneutes erfindungsgemäßes Anlegen einer Schwingung im Frequenzbereich von 20 Hz bis 20 MHz mit einer Amplitude von 1 µm bis 5 mm verarbeitet werden.

Bei dem erfindungsgemäßen Verfahren zum Anmischen geht man so vor, daß die Scherung zwischen zwei Werkstücken auf die Komponenten übertragen wird. Dieses kann z. B. so geschehen, daß ein vibrierender Spatel eingesetzt wird und als Gegenstück wiederum ein Block oder eine Glasplatte vorhanden ist. Unter den vibrierenden Bewegungen z. B. des Spatels verflüssigt sich das Material und läßt sich entsprechend gut vermischen. Eine andere Möglichkeit ist aber auch, daß man einen vibrierenden, löffelähnlichen Körper hat, in dem man mit einem handelsüblichen feststehenden Spatel die Materialien vermischen kann. Auch eine vibrierende Platte mit einem handelsüblichen Spatel ist ein mögliches System. Es ist auch möglich, beide Werkstücke, also z.B. Spatel und Block, gleichzeitig in Vibration zu versetzen.

Eine weitere Ausführungsform des erfindungsgemäßen Verfahrens ist das Vermischen in einem dynamischen oder statischen Mischer. Hierbei kann die Vibration vorteilhafterweise entweder auf die Wandung der Kanüle oder auf die Mischwendel angewandt werden. Hierbei ist es möglich, auch so hochviskose Massen miteinander homogen zu vermischen, die ohne Vibration nicht ohne Zerstörung des Werkstücks durch diese Anordnung hindurchgepreßt werden können. Diese Ausführungsform ist besonders gut geeignet für hochviskose dentale Abformmassen oder Klebstoffe oder Spachtelmassen.

Zur Erzeugung der Vibrationen kann entweder ein eigenes Gerät verwendet werden, welches mittels piezoelektrischer oder aber auch elektromagnetischer Motoren in die entsprechenden Vibrationen versetzt wird, oder es kann ein Einsatz für ein bestehendes Gerät beim Zahnarzt, z. B. Ultraschallscaler oder elektrische Zahnbürste, gefertigt werden, der auf die entsprechenden Gegenstücke paßt und nach Aktivierung die Vibrationen wiederum auf das Material übertragen kann. Die Erzeugung von Vibrationen im erfindungsgemäßen Bereich ist Stand der Technik.

Die erfindungsgemäßen Vorteile sind die folgenden:
1. Verwendungsmöglichkeit für hochviskose, mehrkomponentige Zementierungsmaterialien analog zu DE-OS 40 32 505, die aber jetzt zusätzlich einen zweiten Härtungsmechanismus enthalten können. Hierdurch ist Aushärtung in Schattenzonen möglich.
2. Luftblasenfreies Anmischen ist erstmals bei mehrkomponentigen Systemen möglich. Mit hochfrequenten Vibrationen im genannten Bereich lassen sich Luftblasen auch aus hochviskosen Materialien vollständig entfernen. Hierdurch wird die Härte des Materials positiv beeinflußt, zusätzlich zum ästhetischen Vorteil, da die Luftblasen immer auch an der Oberfläche "zu sehen" sind.
3. Herstellung von dentalen Präparaten mit sehr hohem Füllstoffanteil, die somit im gehärteten Zustand eine besonders hohe Dauerfestigkeit und Abriebbeständigkeit aufweisen.
4. Minimierung des Polymerisationsschrumpfes, der thermischen Expansion sowie des Abriebs durch den erhöhten Füllstoffanteil.

Nachstehend wird die Erfindung anhand von Beispielen näher erläutert.

### Beispiel 1

Aus 70 Gewichtsteilen Bisacryloxymethyltricyclo-(5.2.1.0^{2,6})-decan und 30 Gewichtsteilen 2,2-Bis-4-(3-methacryloxy-2-hydroxypropoxyphenyl)-propan sowie 1 Gewichtsteil p-Chlorbenzoylperoxid wird eine homogene Lösung 1 gemischt.

Mit den gleichen Gewichtsteilen Monomeren sowie 1 Gewichtsteil p-N,N-diethylaminotoluidin sowie 3 Gewichtsteilen N,N-dimethylaminoethylmethacrylat und 0,3 Gewichtsteilen Campherchinon wird eine homogene Lösung 2 gemischt.

21 Gewichtsteile der Lösung 1 werden mit 55 Gewichtsteilen silanisiertem, zahnähnlich eingefärbten Quarz mit einer mittleren Korngröße von 1,5 µm sowie 5 Gewichtsteilen silanisierter pyrogener Kieselsäure mit einer mittleren Korngröße von 0,04 µm sowie 19 Gewichtsteilen silanisiertem Yttriumfluorid mit einer mittleren Korngröße von 1 µm zu einer homogenen Paste 1 (Katalysatorpaste) verknetet.

21 Gewichtsteile der Lösung 2 werden mit den gleichen Gewichtsteilen Quarz, pyrogener Kieselsäure sowie Yttriumfluorid zu einer homogenen Paste 2 (Basispaste) verknetet.

Katalysator- und Basispaste lassen sich mit herkömmlichen Mitteln wie Spatel/Block, Mischkapseln o. ä. nicht homogen miteinander vermischen.

0,5 g Katalysatorpaste werden mit 0,5 g Basispaste in einen löffelähnlichen Aufsatz für eine elektrische Zahnbürste gegeben. Mit einem handelsüblichen Kunststoffspatel läßt sich das Material nicht vermischen, die Viskosität ist so hoch, daß selbst ein Verkneten nicht möglich erscheint. Nach Inbetriebnahme der elektrischen Zahnbürste vibriert der löffelähnliche Aufsatz mit einer Frequenz von 50 Hz und einer Auslenkung von 0,8 mm. Die Materialien lassen sich in diesem Zustand augenblicklich sehr schön vermischen und ergeben ein homogenes vermischtes Endprodukt. Sofort nach Abstellen der Vibrationen hat die angemischte Paste wieder eine hohe Viskosität, die das Material gut modellierbar macht.

Die Paste hat eine Verarbeitungszeit von 7 1/2 Minuten und bindet innerhalb von 15 Minuten ab (23 °C). Die Druckfestigkeit des gehärteten Materials beträgt nach der Aushärtung im Dunkeln 350 MPa, wird der Druckfestigkeitskörper beidseitig noch mit einer handelsüblichen dentalen Beleuchtungseinheit (Elipar Visio, ESPE) für 20 sec. bestrahlt, erhält man eine Druckfestigkeit von 400 MPa. Die Oberflächenhärte des Materials beträgt sowohl dunkel als hell ausgehärtet 240 MPa.

Das vorstehende Beispiel zeigt, daß es erst mit dem erfindungsgemäßen Verfahren möglich ist, eine Vermischung von Basis- und Katalysatorpaste zu erreichen und so zu einem Material zu kommen, welches sowohl die hohen Viskositätseigenschaften aufweist, als auch nach Vermischung mit dem erfindungsgemäßen Verfahren hochwertige physikalische Eigenschaften aufweist. Das Material ist nach der Vermischung absolut blasenfrei, d. h. die bei dünnflüssigen Zementen normalerweise eingemischten Luftblasen wurden durch die Vibration vollständig entfernt. Die Viskosität erniedrigt sich durch die eingebrachten Vibrationen ca. um den Faktor 10, wie sich anhand von einer Filmstärkemessung zeigen läßt. Füllt man ca. 500 mg Basispaste zwischen 2 Glasplatten und belastet diese infolge mit einem Gesamtbelastungsgewicht von 15 kp, so erhält man nach einer Meßzeit von 3 Minuten eine Filmstärke von 110 µm. Führt man die Messung aus wie oben, setzt jedoch die Glasplatten mit Hilfe der im Beispiel aufgeführten elektrischen Zahnbürste in Vibrationen, so erhält man eine Filmstärke von 10 µm. D. h. mit dem erfindungsgemäßen Verfahren ist es möglich, auch so hochfeste Pasten zu Zementierungszwecken zu verwenden, die normalerweise Filmstärken weit über den geforderten 25 µm aufweisen. Durch die eingebrachten Vibrationen erniedrigt sich die Filmstärke um den Faktor 10 von 110 µm auf ca. 10 µm.

### Beispiel 2

Die beiden Pasten von Beispiel 1 werden in gleichen Volumina auf einen handelsüblichen Mischblock (oberflächig gewachstes Papier) gegeben. Anschließend wird mit einem Spatel, welcher an ein handelsübliches Ultraschallgerät (Cavitron, Firma Dentsply) angebracht ist, miteinander vermischt. Nach Einschalten des Ultraschallgerätes (Frequenz ca. 28 kHz, Amplitude 0,05 mm) lassen sich die Materialien leicht miteinander vermischen, wobei nach dem Vermischen keinerlei Luftblasen zu sehen sind. Nach Abschalten des Ultraschallgerätes hat die angemischte Paste sofort wieder die ursprüngliche hohe Viskosität. Die so gemischten Materialien haben physikalische Eigenschaften wie in Beispiel 1 beschrieben.

### Beispiel 3

Aus 70 Gewichtsteilen Bisacryloxymethyltricyclo-(5.2.1.^{2,6})-decan und 30 Gewichtsteilen 2,2-Bis-4-(3-methacryloxy-2-hydroxypropoxy)phenylpropan (Bis-GMA), 7 Gewichtsteilen silanisierter pyrogener Kieselsäure, 0,3 Gewichtsteilen Campherchinon, 3 Gewichtsteilen N,N-Dimethylaminomethylmethacrylat und 110 Gewichtsteilen Yttriumfluorid als röntgenopakem Füllstoff wird eine Vormischung geknetet.

5,96 g dieser Vormischung werden mit einer möglichst großen Menge an silanisiertem und zahnähnlich pigmentierten Quarz (mittlere Korngröße ca. 6 µm) verknetet. Mit einem herkömmlichen Planetenkneter lassen sich maximal 16 g Füllstoff einkneten. Setzt man den Knetertopf jedoch auf eine vibrierende Platte, welche mit einer Amplitude von 0,5 mm und einer Frequenz von 50 Hz vibriert, lassen sich weitere 6 g des Quarzes einkneten. Nach Abstellen der Vibration ist ein Kneten nicht mehr möglich und das Material hat eine feste Viskosität und läßt sich unter Druck nicht nennenswert verformen.

Nimmt man diese Paste jedoch mit einem vibrierenden Spatel, wie er im Beispiel 2 beschrieben ist, auf, so läßt sich das Material beim Anwender wiederum problemlos in eine Kavität einfüllen und man erhält auf diese Art ein extrem hochgefülltes Composite mit extrem niedriger thermischer Expansion, extrem niedrigem Abrieb sowie geringem Polymerisationsschrumpf. Das Material ist zudem hervorragend mit den vibrierenden Instrumenten verarbeitbar, wobei besonders vorteilhaft die feste Konsistenz nach Abstellen der Vibration ist, so daß sich Überschüsse und Ränder im nichtpolymerisierten Zustand perfekt gestalten lassen.

### Beispiel 4

Aus 70 Gewichtsteilen Bisacryloxymethyltricyclo-(5.2.1.0^{2,6})-decan und 30 Gewichtsteilen 2,2-Bis-4-(3-methacryloxy-2-hydroxypropoxyphenyl)-propan, 0,3 Gewichtsteilen Campferchinon und 3 Gewichtsteilen N,N-Dimethylaminoethylmethacrylat wird eine homogene Lösung gemischt.

21 Gewichtsteile dieser Lösung werden mit 55 Gewichtsteilen silanisiertem, zahnähnlich eingefärbtem Quarz mit einer mittleren Kornverteilung von 1,5 µm sowie 5 Gewichtsteilen silanisierter, pyrogener Kieselsäure mit einer mittleren Kornverteilung von 0,04 µm sowie 19 Gewichtsteilen silanisiertem Yttriumfluorid mit einer mittleren Kornverteilung von 1 µm zu einer homogenen Paste verknetet. Die Paste hat eine hochviskose pastöse Konsistenz, welche sich "per se" nicht zum Zementieren eignet.

Mit der so hergestellten Composite-Paste wird eine Inlaykavität vollständig aufgefüllt. Anschließend wird das vorgefertigte Composite Inlay (hergestellt aus dem gleichen Compositepastenmaterial, jedoch bereits vollständig durchgehärtet) in die mit der Paste gefüllte Kavität eingedrückt. Anschließend wird mit einem "Ultrasonic Scaler" (10 000 Hz) (Cavitron, Firma Dentsply) und abgestellter Wasserkühlung mit leichtem Druck auf die Inlayoberfläche des Inlays in die gefüllte Kavität versenkt. Durch den Ultraschall verflüssigt sich die Compositepaste dermaßen, daß sämtliche Überschüsse aus dem Zementierungsspalt hervorquellen und das Inlay bis zur vollständigen Einpassung in die Kavität eingedrückt ist. Die Überschüsse werden anschließend mit einer Sonde und Zahnseide entfernt, was aufgrund der hohen Viskosität der Paste wiederum sehr einfach geht. Anschließend wird mit allseitiger Belichtung über 60 sec. mit einem handelsüblichen dentalen Bestrahlungsgerät (Elipar, Firma ESPE) der Zement auspolymerisiert. Anschließend werden die Zementoberfläche und das Inlay poliert. Es sind keine Übergänge erkennbar, das Inlay paßt hervorragend.

Führt man das Verfahren mit dem gleichen Material an dem gleichen Inlay, jedoch ohne Einwirkung von Ultraschall aus, so läßt sich das Inlay gar nicht vollständig in die Kavität einführen. Es sind Übergänge von der Zahnoberfläche zum Inlay erkennbar, die mit einer Sonde leicht dedektierbar sind.

### Messung der Filmstärke:

Zwischen eine plane Metallplatte und eine Glasplatte werden je 100 mg des oben beschriebenen Composites aufgegeben und gleichmäßig auf eine Ausgangsschichtstärke von 500 µm verteilt. Anschließend wird mit der Oberfläche des Mittelteils des oben genannten "Ultrasonic Scalers" auf die Oberseite der Glasplatte ein Druck von jeweils 0,5 kP aufgebracht. Der Druck wird für je 10 sec. belassen, je einmal mit eingeschalteter und mit ausgeschalteter Vibration. Bei eingeschaltetem Ultraschallgerät erzielt man eine Filmstärke von 10 µm. Ohne eingeschaltete Ultraschalleinheit ist die Filmstärke 400 µm. Auch bei Einwirkung von höheren Drücken (z. B. 10 kP) über längere Zeiten (z. B. 30 sec.) wird eine Filmstärke von 50 µm nicht unterschritten.

## Patentansprüche

1. Verfahren zur Herstellung von Dentalmassen, die ein Bindemittel und einen hohen Anteil an Füllstoffen mit einer mittleren (Gewichtsmittel) Korngröße von <50 µm enthalten, wobei ihr Füllstoffanteil so hoch ist, daß sie für den vorgesehenen Verwendungszweck aufgrund ihrer hohen Viskosität nicht einsetzbar sind, dadurch gekennzeichnet, daß man die Füllstoffe mit dem Bindemittel unter Einwirkung einer Schwingung im Frequenzbereich von 20 Hz bis 50 kHz mit einer Amplitude von 1 µm bis 5 mm vermischt.

2. Verfahren zum Vermischen von mehrkomponentigen Dentalmassen zu hochgefüllten, hochviskosen Mischprodukten, wobei die Komponenten zwischen zwei Werkstücken durch Scherbewegungen vermischt werden, dadurch gekennzeichnet, daß mindestens eines der Werkstücke in eine Schwingung im Frequenzbereich von 20 Hz bis 50 kHz mit einer Amplitude von 1 µm bis 5 mm versetzt wird.

3. Verfahren nach Anspruch 1 oder 2, dadurch gekennzeichnet, daß es sich bei den Dentalmassen um hochgefüllte (Meth)acrylate handelt.

4. Verfahren nach Anspruch 3, dadurch gekennzeichnet, daß die hochgefüllten (Meth)acrylate 70 bis 95 Gew.-% Füllstoffe enthalten.

5. Verfahren nach Anspruch 2, dadurch gekennzeichnet, daß ein Werkstück ein vibrierender Spatel und das andere Werkstück ein Block oder eine Glasplatte ist.

6. Verfahren nach Anspruch 2, dadurch gekennzeichnet, daß ein Werkstück ein vibrierender, löffelähnlicher Körper und das andere Werkstück ein handelsüblicher nicht-vibrierender Spatel ist.

7. Verwendung eines vibrierenden Werkstücks zur Verarbeitung von aushärtbaren Dentalmassen, die ein Bindemittel und einen hohen Anteil an Füllstoffen mit einer mittleren (Gewichtsmittel) Korngröße von <50 µm enthalten, wobei ihr Füllstoffanteil so hoch ist, daß sie für den vorgesehenen Verwendungszweck aufgrund ihrer hohen Viskosität ohne Anwendung von Ultraschall nicht einsetzbar sind, dadurch gekennzeichnet, daß das Werkstück in eine Schwingung im Frequenzbereich von 20 Hz bis 50 kHz mit einer Amplitude von 1 µm bis 5 mm versetzt wird.

8. Verwendung von aushärtbaren Dentalmassen, die ein Bindemittel und einen hohen Anteil an Füllstoffen mit einer mittleren (Gewichtsmittel) Korngröße von <50 µm enthalten, wobei ihr Füllstoffanteil so hoch ist, daß sie für den vorgesehenen Verwendungszweck aufgrund ihrer hohen Viskosität ohne Anwendung von Ultraschall nicht einsetzbar sind, zur Herstellung von dentalen Restaurationen und Befestigung von Inlays, Onlays, Verblendschalen mittels hochfrequenter Vibrationen im Frequenzbereich von 20 Hz bis 50 kHz mit einer Amplitude von 1 bis 5 mm.

9. Verwendung nach Anspruch 8, dadurch gekennzeichnet, daß die Vibration durch ein Ultraschallgerät erzeugt wird.

10. Verwendung nach Anspruch 8 oder 9, dadurch gekennzeichnet, daß die Vibrationsfrequenz mindestens 10 000 Hz beträgt.

11. Verwendung nach Anspruch 9, dadurch gekennzeichnet, daß die Dentalmasse folgende Komponenten enthält:
a) 60 - 95 Gew.-% anorganische Füllstoffe,
b) 4 - 39,99 Gew.-% ethylenisch ungesättigte polymerisierbare Monomere und/oder Polymere
c) 0,01 - 3 Gew.-% Photoinitiatoren
d) sowie ggf. Aktivatoren, Initiatoren für die Initiierung einer Redox-Polymerisierung sowie Pigmente, röntgenopake Zusatzstoffe und/oder Thixotropiehilfsmittel.

12. Verwendung nach Anspruch 11, dadurch gekennzeichnet, daß der Anteil der anorganischen Füllstoffe 70 - 90 Gew.-%, der Anteil der ethylenisch ungesättigten Monomeren und/oder Polymeren 9 - 29,9 Gew.-% und der Anteil der Photoinitiatoren 0,1 - 2 Gew.-% betragen.

13. Verwendung nach Anspruch 8 bis 12, dadurch gekennzeichnet, daß die anorganischen Füllstoffe eine mittlere Korngröße von ≦ 5 µm aufweisen und eine obere Korngrenze von 25 µm aufweisen.

14. Verwendung nach Anspruch 13, dadurch gekennzeichnet, daß 5 - 25 Gew.-% der Füllstoffe eine mittlere Korngröße von 0,02 - 0,06 µm und 65 - 85 Gew.-% die Füllstoffe eine mittlere Korngröße von 1 -5 µm aufweisen.

15. Verwendung nach Anspruch 11, dadurch gekennzeichnet, daß es sich bei der Komponente b) um Acryl- oder Methacrylsäureester ein- oder mehrwertiger Alkohole handelt.

16. Verwendung nach Anspruch 9, dadurch gekennzeichnet, daß zwischen dem Ultraschallgerät und dem zu befestigenden Teil eine Zwischenschicht vorgesehen ist.

## Claims

1. Method for preparing curable dental compositions containing a binder and a high proportion of fillers having a mean (weight average) grain size of < 50 µm, their filler proportion being so high that they cannot be used for the intended purpose because of their high viscosity, characterized in that the fillers are mixed with the binder under the action of oscillations in the frequency range of 20 Hz to 50 kHz with an amplitude of 1 µm to 5 mm.

2. Method of mixing multicomponent dental compositions to give highly filled high-viscosity mixed products, the components being mixed between two workpieces by shearing motions, characterized in that at least one of the workpieces is set into oscillation in the frequency range of 20 Hz to 50 kHz with an amplitude of 1 µm to 5 mm.

3. Method according to claim 1 or 2, characterized in that the dental compositions are highly filled (meth)acrylates.

4. Method according to claim 3, characterized in that the highly filled (meth)acrylates contain 70 to 95 % by weight fillers.

5. Method according to claim 2, characterized in that one workpiece is a vibrating spatula and the other workpiece a block or a glass plate.

6. Method according to claim 2, characterized in that one workpiece is a vibrating spoon-like body and the other workpiece is a commercially available non-vibrating spatula.

7. Use of a vibrating workpiece for working dental compositions containing a binder and a high proportion of fillers having a mean (weight average) grain size of < 50 µm, their filler proportion being so high that they cannot be used for the intended purpose because of their high viscosity, characterized in that the workpiece is set into oscillation in the frequency range of 20 Hz to 50 kHz with an amplitude of 1 µm to 5 mm.

8. Use of curable dental compositions containing a binder and a high proportion of fillers having a mean (weight average) grain size of < 50 µm, their filler proportion being so high that they cannot be used for the intended purpose without using ultrasound because of their high viscosity, for cementing inlays, onlays, veneers by means of high-frequency vibrations in the frequency range of 20 Hz to 50 kHz with an amplitude of 1 µm to 5 mm.

9. Use according to claim 8, characterized in that the vibration is generated by an ultrasonic device.

10. Use according to claim 8 or 9, characterized in that the frequency is at least 10,000 Hz.

11. Use according to claim 9, characterized in that the filling composition contains the following components:
a) 60 - 95 % by weight inorganic fillers;
b) 4 - 39,99 % by weight ethylenically unsaturated polymerisable monomers and/or polymers;
c) 0,01 - 3 % by weight photoinitiators;
d) and possibly activators, initiators for the initiation of a redox polymerisation, as well as pigments, X-ray-opaque additives and/or thixotropy aids.

12. Use according to claim 11, characterized in that the amount of inorganic fillers is 70 - 90 % by weight, the amount of ethylenically unsaturated polymerisable monomers and/or polymers is 9 - 29,9 % by weight and the amount of photoinitiators is 0,1 - 2 % by weight.

13. Use according to any of claims 8 to 12, characterized in that the inorganic fillers have a mean particle size of ≦ 5 µm and an upper grain limit of 25 µm.

14. Use according to claim 13, characterized in that 5 - 25 % by weight of the fillers have a mean particle size of 0,02 - 0,06 µm and 65 - 85 % by weight of the fillers have a mean particle size of 1 - 5 µm.

15. Use according to claim 11, characterized in that component b) is an acrylic or methacrylic acid ester of a monohydric or polyhydric alcohol.

16. Use according to claim 9, characterized in that an intermediate layer is provided between the ultrasonic device and the part to be secured.

## Revendications

1. Procédé de préparation de compositions dentaires qui contiennent un liant et une proportion élevée de charges ayant une grosseur de grains moyenne (moyenne en poids) inférieure à 50 µm, et dont la proportion de charges est si élevée qu'elles ne sont pas utilisables pour l'application prévue en raison de leur grande viscosité, caractérisé en ce que l'on mélange les charges avec le liant sous l'action de vibrations situées dans un intervalle de fréquences de 20 Hz à 50 kHz avec une amplitude de 1 µm à 5 mm.

2. Procédé de mélange de compositions dentaires à plusieurs constituants pour former des produits mélangés à haute teneur en charges, très visqueux, les constituants étant mélangés par des mouvements de cisaillement entre deux pièces, caractérisé en ce qu'au moins l'une des pièces est animée d'une vibration dans un intervalle de fréquences de 20 Hz à 50 kHz avec une amplitude de 1 µm à 5 mm.

3. Procédé selon la revendication 1 ou 2, caractérisé en ce que les compositions dentaires sont des (méth)acrylates à haute teneur en charges.

4. Procédé selon la revendication 3, caractérisé en ce que les (méth)acrylates à haute teneur en charges contiennent 70 à 95 % en masse de charges.

5. Procédé selon la revendication 2, caractérisé en ce que l'une des pièces est une spatule vibrante et l'autre est un bloc ou une plaque en verre.

6. Procédé selon la revendication 2, caractérisé en ce que l'une des pièces est un corps vibrant en forme de cuiller et l'autre est une spatule non vibrante courante dans le commerce.

7. Utilisation d'une pièce vibrante pour façonner des compositions dentaires durcissables qui contiennent un liant et une proportion élevée de charges ayant une grosseur de grains moyenne (moyenne en poids) inférieure à 50 µm, et dont la proportion de charges est si élevée qu'elles ne sont pas utilisables sans l'emploi d'ultrasons pour l'application prévue en raison de leur grande viscosité, caractérisée en ce que la pièce est animée de vibrations situées dans un intervalle de fréquences de 20 Hz à 50 kHz avec une amplitude de 1 µm à 5 mm.

8. Utilisation de compositions dentaires durcissables qui contiennent un liant et une proportion élevée de charges ayant une grosseur de grains moyenne (moyenne en poids) inférieure à 50 µm, et dont la proportion de charges est si élevée qu'elles ne sont pas utilisables sans l'emploi d'ultrasons pour l'application prévue en raison de leur grande viscosité, pour la préparation de reconstitutions dentaires et la fixation d'incrustations en profondeur, d'incrustations de surface, de couronnes de revêtement, à l'aide de vibrations de haute fréquence comprises dans un intervalle de fréquences de 20 Hz 50 kHz avec une amplitude de 1 µm à 5 mm.

9. Utilisation selon la revendication 8, caractérisée en ce que la vibration est obtenue à l'aide d'un appareil à ultrasons.

10. Utilisation selon la revendication 8 ou 9, caractérisée en ce que la fréquence des vibrations est d'au moins 10 000 Hz.

11. Utilisation selon la revendication 9, caractérisée en ce que la composition dentaire contient les constituants suivants:
a) 60 à 95 % en masse de charges inorganiques,
b) 4 à 39,99 % en masse de monomères polymérisables et/ou polymères à insaturation éthylénique
c) 0,01 à 3 % en masse de photoamorceurs
d) et éventuellement des activateurs, des amorceurs pour amorcer une polymérisation redox et des pigments, des additifs opaques aux rayons X et/ou des agents thixotropes.

12. Utilisation selon la revendication 11, caractérisée en ce que la proportion de charges inorganiques est de 70 à 90 % en masse, la proportion de monomères et/ou polymères à insaturation éthylénique est de 9 à 29,9 % en masse et la proportion de photoamorceurs est de 0,1 à 2 % en masse.

13. Utilisation selon les revendications 8 à 12, caractérisée en ce que les charges inorganiques ont une grosseur de grains moyenne inférieure ou égale à 5 µm et une limite supérieure de la grosseur des grains de 25 µm.

14. Utilisation selon la revendication 13, caractérisée en ce que 5 à 25 % en masse des charges ont une grosseur de grains moyenne de 0,02 à 0,06 µm et 65 à 85 % en masse des charges ont une grosseur de grains moyenne de 1 à 5 µm.

15. Utilisation selon la revendication 11, caractérisée en ce que le constituant b) est un ester d'acide acrylique ou méthacrylique de mono- ou polyalcools.

16. Utilisation selon la revendication 9, caractérisée en ce qu'une couche intermédiaire est prévue entre l'appareil à ultrasons et la partie à fixer.
